# EUROPÄISCHE PATENTSCHRIFT

(11) **EP 1 341 538 B1**
(45) Veröffentlichungstag und Bekanntmachung des Hinweises auf die Patenterteilung: **23.12.2009**
(21) Anmeldenummer: 01982446.5
(22) Anmeldetag: 23.10.2001
(51) Int. Cl.: A61K 31/46, A61K 31/55, A61P 11/00, A61P 11/02

(54) **ARZNEIMITTELKOMPOSITIONEN AUS TIOTROPIUMSALZEN UND EPINASTIN ZUR THERAPIE VON ATEMSWEGSERKRANKUNGEN**
MEDICAMENT COMPOSITIONS WITH SALTS OF TIOTROPIUM AND EPINASTIN FOR THE THERAPY OF RESPIRATORY DISEASES
COMPOSITIONS DES MEDICAMENTS AVEC DES SELS DU TIOTROPIUM ET EPINASTIN POUR LA THERAPIE DES MALADIES RESPIRATOIRES

(30) Priorität: 31.10.2000 DE 10054042; 10.08.2001 DE 10138272
(43) Veröffentlichungstag der Anmeldung: 10.09.2003
(73) Patentinhaber: Boehringer Ingelheim Pharma GmbH & Co.KG, 55216 Ingelheim am Rhein (DE)
(72) Erfinder: PAIRET, Michel, 55442 Stromberg (DE); PIEPER, Michael, Paul, 88400 Biberach (DE); MEADE, Christopher, John, Montague, 55411 Bingen (DE); SCHMELZER, Christel, 55218 Ingelheim am Rhein (DE)
(74) Vertreter: Weymann, Markus
(86) Internationale Anmeldenummer: PCT/EP2001/012510
(87) Internationale Veröffentlichungsnummer: WO 2002/036163

(56) Entgegenhaltungen:
- WO-A-97/46243
- WO-A-98/41256
- DE-A- 4 203 306
- US-A- 5 824 669
- NACLERIO R M: "Optimizing treatment options." CLINICAL AND EXPERIMENTAL ALLERGY, Bd. 28, Nr. SUPPL. 6, Dezember 1998 (1998-12), Seiten 54-59, XP001078789 ISSN: 0954-7894
- SIMONS F E R ET AL: "OPTIMUM PHARMACOLOGICAL MANAGEMENT OF CHRONIC RHINITIS" DRUGS, Bd. 38, Nr. 2, 1989, Seiten 313-331, XP001078899 ISSN: 0012-6667
- MON F ET AL: "AEROSOL THERAPY IN ASTHMA" REVUE DES MALADIES RESPIRATOIRES, Bd. 6, Nr. 3, 1989, Seiten 189-200, XP001078722 ISSN: 0761-8425
- PAVIA D ET AL: "Preliminary data from phase II studies with Respimat, a propellant-free soft mist inhaler." JOURNAL OF AEROSOL MEDICINE: THE OFFICIAL JOURNAL OF THE INTERNATIONAL SOCIETY FOR AEROSOLS IN MEDICINE. UNITED STATES 1999, Bd. 12 Suppl 1, 1999, Seiten S33-S39, XP001078739 ISSN: 0894-2684
- PATENT ABSTRACTS OF JAPAN vol. 1999, no. 02, 26. Februar 1999 (1999-02-26) & JP 10 298107 A (TAISHO PHARMACEUT CO LTD), 10. November 1998 (1998-11-10)

## Beschreibung

Die vorliegende Erfindung betrifft neuartige Arzneimittelkompositionen auf der Basis von Tiotropiumsalzen und dem Antihistaminikum Epinastin, Verfahren zu deren Herstellung sowie deren Verwendung bei der Therapie von Atemwegserkrankungen.

### Beschreibung der Erfindung

Die vorliegende Erfindung betrifft neuartige Arzneimittelkompositionen auf der Basis von Tiotropiumsalzen und dem Antihistaminikum Epinastin, Verfahren zu deren Herstellung sowie deren Verwendung bei der Therapie von Atemwegserkrankungen.

Sowohl Anticholinergika als auch Antihistaminika sind bekanntermaßen bei der Therapie von Atemwegserkrankungen einsetzbar. So offenbaren beispielsweise Naclerio (Clinical and Experimental Allergy, Vol 28, Suppl. 6, 1998, 54-59) oder auch Simons (Drugs, Vol 38, No. 2, 1989, 313-331) mögliche Therapien von beispielsweise Rhinitis mittels Ipratropiumbromid und H1-Rezeptor-Antagonisten, gegebenenfalls in Kombination mit Corticosteroiden. Therapieansätze mit beispielsweise Tiotropiumbromid werden allerdings nicht offenbart.

Überraschenderweise kann ein unerwartet vorteilhafter therapeutischer Effekt, insbesondere ein synergistischer Effekt bei der Behandlung von Erkrankungen der oberen und unteren Atemwege, insbesondere bei der Behandlung allergischer oder nicht allergischer Rhinitis beobachtet werden, wenn ein oder mehrere, bevorzugt ein Tiotropiumsalz gemeinsam mit dem Antihistaminikum Epinastin zur Anwendung gelangen.

Die vorstehend genannten Effekte werden sowohl bei gleichzeitiger Applikation innerhalb einer einzigen Wirkstoffformulierung als auch bei sukzessiver Applikation der beiden Wirkstoffe in getrennten Formulierungen beobachtet. Erfindungsgemäß bevorzugt ist die gleichzeitige Applikation der beiden Wirkstoffbestandteile in einer einzigen Formulierung.

Im Rahmen der vorliegenden Erfindung werden unter Tiotropiumsalzen **1** Salze verstanden, die als Kation Tiotropium, dem pharmakologisch wirksamen Bestandteil, enthalten, Im Rahmen der vorliegenden Patentanmeldung ist eine Bezugnahme auf vorstehendes Kation durch Verwendung der Bezeichnung **1'** anzusehen. Eine Bezugnahme auf Verbindungen **1** schließt naturgemäß eine Bezugnahme auf den Bestandteil **1'** (Tiotropium) mit ein.

Unter den im Rahmen der vorliegenden Erfindung einsetzbaren Salzen **1** sind die Verbindungen zu verstehen, die neben Tiotropium als Gegenion (Anion) Chlorid, Bromid, Iodid, Methansulfonat, para-Toluolsulfonat oder Methylsulfat enthalten. Im Rahmen der vorliegenden Erfindung sind von allen Salzen **1** das Methansulfonat, Chlorid, Bromid oder Iodid bevorzugt, wobei dem Methansulfonat oder dem Bromid besondere Bedeutung zukommt. Von erfindungsgemäß herausragender Bedeutung ist als **Salz 1** das Tiotropiumbromid.

Im Rahmen der vorliegenden Erfindung wird unter Antihistaminikum (im Folgenden **2**) Epinastin verstanden.

Eine Bezugnahme auf **Epinastin 2** schließt im Rahmen der vorliegenden Erfindung eine Bezugnahme auf dessen gegebenenfalls existierende pharmakologisch verträgliche Säureadditionssalze ein.

Unter den physiologisch verträglichen Säureadditionssalzen, die **von 2** gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind die Salze der **Verbindungen 2** die ausgewählt sind aus der Gruppe bestehend aus Acetat, Hydrochlorid, Hydrobromid, Sulfat, Phosphat, und Methansulfonat. Besonders bevorzugt sind in diesem Zusammenhang Hydrochloride und Hydrobromide. Erfindungsgemäß kommt dem Epinastinhydrochlorid eine herausragende Bedeutung zu.

Die Applikation der erfindungsgemäßen Arzneimittelkombinationen aus **1** und **2** erfolgt vorzugsweise auf inhalativem Wege oder als nasale Administration. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch Inhalationsaerosole, die beispielsweise HFA134a (auch TG134a genannt), HFA227 (auch TG227 genannt) oder deren Gemisch als Treibgas enthalten. Die inhalative Applikation kann ferner mittels geeigneter Lösungen der Arzneihittelkombination bestehend aus **1** und **2** erfolgen.

Erfolgt die Applikation der Arzneimittelkombination bestehend aus **1** und **2** nasal, können geeignete Lösungen, die mittels entsprechender Pumpen appliziert werden, zum Einsatz kommen. Alternativ dazu kann eine nasale Anwendung auch durch Applikation geeigneter Pulver erfolgen.

Ein Aspekt der vorliegenden Erfindung betrifft dementsprechend ein Arzneimittel, welches eine Kombination aus **1** und **2** enthält.

Ein weiterer Aspekt der vorliegenden Erfindung betrifft ein Arzneimittel, welches ein oder mehrere Salze **1** und ein oder mehrere Verbindungen **2**, gegebenfalls in Form ihrer Solvate oder Hydrate enthält. Auch hierbei können die Wirkstoffe entweder gemeinsam in einer einzigen Darreichungsform oder in zwei getrennten. Darreichungsformen enthalten sein. Erfindungsgemäß bevorzugt sind Arzneimittel, die die Wirkstoffe **1** und **2** in einer einzigen Darreichungsform enthalten.

Ein weiterer Aspekt der vorliegenden Erfindung betrifft ein Arzneimittel, welches neben therapeutisch wirksamen Mengen von **1** und **2** einen pharmazeutisch verträglichen Trägerstoff enthält. Ein weiterer Aspekt der vorliegenden Erfindung betrifft ein Arzneimittel, welches neben therapeutisch wirksamen Mengen von **1** und **2** keinen pharmazeutisch verträglichen Trägerstoff enthält

Die vorliegende Erfindung betrifft ferner die Verwendung von **1** und **2** zur Herstellung eines therapeutisch wirksame Mengen von **1** und **2** enthaltenden Arzneimittels zur Behandlung von Erkrankungen der oberen und unteren Atemwege, insbesondere zur Behandlung von allergischer oder nicht allergischer Rhinitis

Die vorliegende Erfindung zielt ferner auf die simultane oder sukzessive Verwendung therapeutisch wirksamer Dosen der Kombination vorstehender Arzneimittel **1** und **2** zur Behandlung Erkrankungen der oberen und unteren Atemwege, insbesondere zur Behandlung allergischer oder nicht allergischer Rhinitis.

In den erfindungsgemäßen Wirkstoffkombinationen aus **1** und **2** können die **Bestandteile 1** und **2** in Form ihrer Enantiomere, Gemische der Enantiomere oder in Form der Racemate enthalten sein.

Die Verhältnisse, in denen die beiden Wirkstoffe **1** und **2** in die erfindungsgemäßen Wirkstoffkombinationen eingesetzt werden können, sind variabel. Die Wirkstoffe **1** und **2** können gegebenfalls in Form ihrer Solvate oder Hydrate vorliegen. Je nach Wahl der Verbindungen **1** bzw. **2** variieren die im Rahmen der vorliegenden Erfindung einsetzbaren Gewichtsverhältnisse aufgrund des unterschiedlichen Molekulargewichts der verschiedenen Verbindungen sowie aufgrund ihrer unterschiedlichen Wirkstärke. In der Regel können die erfindungsgemäßen Arzneimittelkombinationen die Verbindungen **1** und **2** in Gewichtsverhältnissen enthalten, die in einem Bereich von 1:300 bis 50:1, bevorzugt von 1:250 bis 40:1, liegen. Bei den besonders bevorzugten Arzneimittelkombinationen, die Tiotropiumsalz als Verbindung **1** enthalten, liegen die Gewichtsverhältnisse von **1** zu **2** besonders bevorzugt in einem Bereich, in dem Tiotropium **1'** und **2** in Verhältnissen von 1:150 bis 30:1, ferner bevorzugt von 1:50 bis 20:1 enthalten sind.

Beispielsweise und ohne den Umfang der Erfindung darauf zu beschränken, können bevorzugte erfindungsgemäße Kombinationen aus **1** und **2 Tiotropium 1'** und **Antihistaminikum 2** in den folgenden Gewichtsverhältnissen enthalten:
1:80; 1:79; 1:78; 1:77; 1:76; 1:75; 1:74; 1:73; 1:72; 1:71; 1:70; 1:69; 1:68; 1:67; 1:66; 1:65; 1:64; 1:63 1:62; 1:61; 1:60; 1:59; 1:58; 1:57; 1:56; 1:55; 1:54; 1:53; 1:52; 1:51; 1:50; 1:49; 1:48; 1:47; 1:46; 1:45; 1:44; 1:43; 1:42; 1:41; 1:40; 1:39; 1:38; 1:37; 1:36; 1:35; 1:34; 1:33; 1:32; 1:31; 1:30; 1:29; 1:28; 1:27; 1:26; 1:25; 1:24; 1:23; 1:22; 1:21; 1:20; 1:19; 1:18; 1:17; 1:16; 1:15; 1:14:1:13; 1:12; 1:11; 1:10; 1:9; 1:8; 1:7; 1:6; 1:5; 1:4; 1:3; 1:2; 1:1; 2:1; 3:1; 4:1; 5:1; 6:1; 7:1; 8:1; 9:1; 10:1; 11:1; 12:1; 13:1; 14:1; 15:1; 16:1; 17:1; 18:1; 19:1; 20:1.

Die Anwendung der erfindungsgemäßen Arzneimittel enthaltend die Kombinationen aus **1** und **2** erfolgt üblicherweise so, daß **1** und **2** gemeinsam in Dosierungen von 0,01 bis 10000µg, bevorzugt von 0,1 bis 2000µg, besonders bevorzugt von 1 bis 1500µg, ferner bevorzugt von 50 bis 1200µg pro Einmalgabe enthalten sind. Beispielsweise enthalten erfindungsgemäße Kombinationen aus **1** und **2** eine solche Menge an Tiotropium **1'** und Antihistaminikum **2**, daß die Gesamtdosierung pro Einmalgabe 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg, 1000µg, 1005µg, 1010µg, 1015µg, 1020µg, 1025µg, 1030µg, 1035µg, 1040µg, 1045µg, 1050µg, 1055µg, 1060µg, 1065µg, 1070µg, 1075µg, 1080µg, 1085µg, 1090µg, 1095µg, 1100µg oder ähnliches beträgt. Vorstehend genannte Dosierungsvorschläge pro Einmalgabe sind nicht als auf die explizit angegebenen Zahlenwerte beschränkt anzusehen, sondern dienen nur als beispielhaft offenbarte Dosierungen. Selbstverständlich sind beispielsweise auch Dosierungen, die um o.g. Zahlenwerte in einem Bereich von ca. +/- 2,5µg schwanken, von den vorliegenden exemplarisch erläuterten Werten umfaßt. Bei diesen Dosierungsbereichen können die Wirkstoffe **1'** und **2** in den vorhergehend beschriebenen Gewichtsverhältnissen enthalten sein.

Beispielsweise und ohne den Umfang der Erfindung darauf zu beschränken, können die erfindungsgemäßen Kombinationen aus **1** und **2** eine solche Menge an Tiotropium **1'** und Antihistaminikum **2** enthalten, daß pro Einmalgabe 5µg **1'** und 25µg **2**, 5µg **1'** und 50µg **2**, 5µg **1'** und 100µg **2**, 5µg **1'** und 200µg **2**, 5µg **1'** und 300µg **2**, 5µg **1'** und 400µg **2**, 5µg **1'** und 500µg **2**, 5µg **1'** und 600µg **2**, 5µg **1'** und 700µg **2**, 5µg **1'** und 800µg **2**, 5µg **1'** und 900µg **2**, 5µg **1'** und 1000µg **2**, 10µg **1'** und 25µg **2,** 10µg **1'** und 50µg **2**, 10µg **1'** und 100µg **2**, 10µg **1'** und 200µg **2**, 10µg **1'** und 300µg **2**, 10µg **1'** und 400µg **2**, 10µg **1'** und 500µg **2**, 10µg **1'** und 600µg **2**, 10µg **1'** und 700µg **2**, 10µg **1'** und 800µg **2**, 10µg **1'** und 900µg **2**, 10µg **1'** und 1000µg **2**, 18µg **1'** und 25µg **2**, 18µg **1'** und 50µg **2**, 18µg **1'** und 100µg **2**, 18µg **1'** und 200µg **2**, 18µg **1'** und 300µg **2**, 18µg **1'** und 400µg **2**, 18µg **1'** und 500µg **2**, 18µg **1'** und 600µg **2**, 18µg **1'** und 700µg **2**, 18µg **1'** und 800µg **2**, 18µg **1'** und 900µg **2**, 18µg **1'** und 1000µg **2**, 20µg **1'** und 25µg **2**, 20µg **1'** und 50µg **2**, 20µg **1'** und 100µg **2**, 20µg **1'** und 200µg **2**, 20µg **1'** und 300µg **2**, 20µg **1'** und 400µg **2**, 20µg **1'** und 600µg **2**, 20µg **1'** und 600µg **2**, 20µg **1'** und 700µg **2**, 20µg **1'** und 800µg **2**, 20µg **1'** und 900µg **2**, 20µg **1'** und 1000µg **2**, 36µg **1'** und 25µg **2**, 36µg **1'** und 50µg **2**, 36µg **1'** und 100µg **2**, 36µg **1'** und 200µg **2**, 36µg **1'** und 300µg **2,** 36µg **1'** und 400µg **2,** 36µg **1'** und 500µg **2,** 36µg **1'** und 600µg **2**, 36µg **1'** und 700µg **2**, 36µg **1'** und 800µg **2**, 36µg **1'** und 900µg **2**, 36µg **1'** und 1000µg **2**, 40µg **1'** und 25µg **2**, 40µg **1'** und 50µg **2**, 40µg **1'** und 100µg **2**, 40µg **1'** und 200µg **2,** 40µg **1'** und 300µg **2**, 40µg **1'** und 400µg **2,** 40µg **1'** und 500µg **2** oder 40µg **1'** und 600µg **2,** 40µg **1'** und 700µg **2**, 40µg **1'** und 800µg **2**, 40µg **1'** und 900µg **2**, 40µg **1'** und 1000µg **2** appliziert werden.

Wird als erfindungsgemäß bevorzugte Kombination aus **1** und **2** die Wirkstoffkombination verwendet, in der **1** Tiotropiumbromid bedeutet, entsprechen die vorstehend beispielhaft genannten pro Einmalgabe applizierten Wirkstoffmengen von **1'** und **2** den nachfolgenden pro Einmalgabe applizierten Mengen an **1** und **2:** 6µg **1** und 25µg **2,** 6µg **1** und 50µg **2,** 6µg **1** und 100µg **2**, 6µg **1** und 200µg **2,** 6µg **1** und 300µg **2**, 6µg **1** und 400µg **2**, 6µg **1** und 500µg **2**, 6µg **1** und 600µg **2**, 6µg **1** und 700µg **2**, 6µg **1** und 800µg **2,** 6µg **1** und 900µg **2**, 6µg **1** und 1000µg **2**, 12µg **1** und 25µg **2,** 12µg **1** und 50µg **2,** 12µg **1** und 100µg **2,** 12µg **1** und 200µg **2,** 12µg **1** und 300µg **2,** 12µg **1** und 400µg **2**, 12µg **1** und 500µg **2**, 12µg **1** und 600µg **2**, 12µg **1** und 700µg **2**, 12µg **1** und 800µg **2**, 12µg **1** und 900µg **2**, 12µg **1** und 1000µg **2**, 21,7µg **1** und 25µg **2**, 21,7µg **1** und 50µg **2**, 21,7µg **1** und 100µg **2**, 21,7µg **1** und 200µg **2**, 21,7µg **1** und 300µg **2**, 21,7µg **1** und 400µg **2**, 21,7µg **1** und 500µg **2**, 21,7µg **1** und 600µg **2**, 21,7µg **1** und 700µg **2,** 21,7µg **1** und 800µg **2**, 21,7µg **1** und 900µg **2**, 21,7µg **1** und 1000µg **2**, 24,1 µg **1** und 25µg **2**, 24,1 µg **1** und 50µg **2,** 24,1µg **1** und 100µg **2**, 24,1µg **1** und 200µg **2**, 24,1 µg **1** und 300µg **2,** 24,1µg **1** und 400µg **2**, 24,1µg **1** und 500µg **2**, 24,1µg **1** und 600µg **2**, 24,1µg **1** und 700µg **2,** 24,1µg **1** und 800µg **2**, 24,1µg **1** und 900µg **2**, 24,1 µg **1** und 1000µg **2**, 43,3µg **1** und 25µg **2**, 43,3µg **1** und 50µg **2**, 43,3µg **1** und 100µg **2**, 43,3µg **1** und 200µg **2,** 43,3µg **1** und 300µg **2**, 43,3µg **1** und 400µg **2**, 43,3µg **1** und 500µg **2**, 43,3µg **1** und 600µg **2**, 43,3µg **1** und 700µg **2**, 43,3µg **1** und 800µg **2**, 43,3µg **1** und 900µg **2**_{,} 43,3µg **1** und 1000µg **2,** 48,1µg **1** und 25µg **2**, 48,1µg **1** und 50µg **2,** 48,1µg **1** und 100µg **2**, 48,1µg **1** und 200µg **2**, 48,1 µg **1** und 300µg **2**, 48,1µg **1** und 400µg **2**, 48,1µg **1** und 500µg **2,** 48,1 µg **1** und 600µg **2**, 48,1 µg **1** und 700µg **2**, 48,1µg **1** und 800µg **2**, 48,1µg **1** und 900µg **2** oder 48,1µg **1** und 1000µg **2**.

Wird als erfindungsgemäß bevorzugte Kombination aus **1** und **2** die Wirkstoffkombination verwendet, in der **1** Tiotropiumbromidmonohydrat bedeutet, entsprechen die vorstehend beispielhaft genannten pro Einmalgabe applizierten Wirkstoffmengen von **1'** und **2** den nachfolgenden pro Einmalgabe applizierten Mengen an **1** und **2**: 6,2µg **1** und 25µg **2**, 6,2µg 1 und 50µg **2**, 6,2µg **1** und 100µg **2**, 6,2µg **1** und 200Ng **2**, 6,2µg **1** und 300µg **2**, 6,2µg **1** und 400µg **2**, 6,2µg **1** und 500µg **2**, 6,2µg **1** und 600µg **2**, 6,2µg **1** und 700µg **2,** 6,2µg **1** und 800µg **2**, 6,2µg **1** und 900µg **2**, 6,2µg 1 und 1000µg **2**, 12,5µg **1** und 25µg **2**, 12,5µg **1** und 50Ng **2**, 12,5µg **1** und 100µg **2**, 12,5µg **1** und 200µg **2,** 12,5µg **1** und 300µg **2**, 12,5µg **1** und 400µg **2**, 12,5µg **1** und 500µg **2**, 12,5µg **1** und 600µg **2**, 12,5µg **1** und 700µg **2**, 12,5µg **1** und 800µg **2**, 12,5µg **1** und 900µg **2**, 12,5µg **1** und 1000µg **2**, 22,5µg **1** und 25µg **2,** 22,5µg **1** und 50µg **2,** 22,5µg **1** und 100µg **2,** 22,5µg **1** und 200µg **2,** 22,5µg **1** und 300µg **2**, 22,5µg **1** und 400µg **2**, 22.5µg **1** und 500µg **2**, 22,5µg **1** und 600µg **2**, 22,5µg **1** und 700µg **2**, 22,5µg **1** und 800µg **2**, 22,5µg **1** und 900µg **2**, 22,5µg **1** und 1000µg **2**, 25µg **1** und 25µg **2**, 25µg **1** und 50µg **2**, 25µg **1** und 100µg **2,** 25µg **1** und 200µg **2**, 25µg **1** und 300µg **2**, 25µg **1** und 400µg **2**, 25µg **1** und 500µg **2**, 25µg **1** und 600µg **2**, 25µg **1** und 700µg **2,** 25µg **1** und 800µg **2**, 25µg **1** und 900µg **2,** 25µg **1** und 1000µg **2**, 45µg **1** und 25µg **2**, 45µg **1** und 50µg **2**, 45µg **1** und 100µg **2**, 45µg **1** und 200µg **2**, 45µg **1** und 300µg **2**, 45µg **1** und 400µg **2**, 45µg **1** und 500µg **2**, 45µg **1** und 600µg **2**, 45µg **1** und 700µg **2,** 45µg **1** und 800µg **2**, 45µg **1** und 900µg **2**, 45µg **1** und 1000µg **2**, 50µg **1** und 25µg **2**, 50µg **1** und 50µg **2**, 50µg **1** und 100µg **2**, 50µg **1** und 200µg **2**, 50µg **1** und 300µg **2**, 50µg **1** und 400µg **2**, 50µg **1** und 500µg **2**, 50µg **1** und 600µg **2**, 50µg **1** und 700µg **2**, 50µg **1** und 800µg **2**, 50µg **1** und 900µg **2** oder 50µg 1 und 1000µg **2**.

Die Applikation der erfindungsgemäßen Wirkstoffkombinationen aus **1** und **2** erfolgt bevorzugt auf inhalativem oder nasalem Wege. Hierzu müssen die Bestandteile **1** und **2** in inhalierbaren oder nasalen Darreichungsformen bereitgestellt werden. Als inhalierbare Darreichungsformen kommen Inhalationspulver, treibgashaltige Dosieraerosole oder treibgasfreie Inhalationslösungen in Betracht. Erfindungsgemäße Inhalationspulver enthaltend die Wirkstoffkombination aus **1** und **2** können allein aus den genannten Wirkstoffen oder aus einem Gemisch der genannten Wirkstoffe mit physiologisch verträglichen Hilfsstoffen bestehen. Im Rahmen der vorliegenden Erfindung sind von dem Begriff treibgasfreie Inhaltionslösungen auch Konzentrate oder sterile, gebrauchsfertige Inhalationslösungen umfaßt. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der beschreibung detailliert beschrieben.

### A) Inhalationspulver enthaltend die erfindungsgemäßen Wirkstoffkombinationen aus 1 und 2:

Die erfindungsgemäßen Inhaltionspulver können **1** und **2** entweder allein oder im Gemisch mit geeigneten physiologisch unbedenkliche. Hilfsstoffen enthalten.

Sind die Wirkstoffe **1** und **2** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1** und **2**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen lnhaltionspulver durch Mahlen und Mikronisieren sowie durch abschließendes Mischen der Bestandteile sind aus dem Stand der Technik bekannt. Die erfindungsgemäßen Inhaltionspulver können entweder in Form einer einzigen Pulvermischung; die sowohl **1** als auch **2** enthält oder in Form von separaten Inhalationspulvern, die lediglich **1** und **2** enthalten bereitgestellt und appliziert werden.

Die erfindungsgemäßen Inhalationspulver können mittels aus dem Stand der Technik bekannten Inhalatoren appliziert werden. Erfindungsgemäße Inhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1** und **2** physiologisch unbedenkliche Hilfsstoff enthalten, allerdings in Kapseln abgefüllt (zu sogenannten Inhaletten), die in Inhalatoren wie beispielsweise in der WO 94/28958 beschrieben, zur Anwendung gelangen.

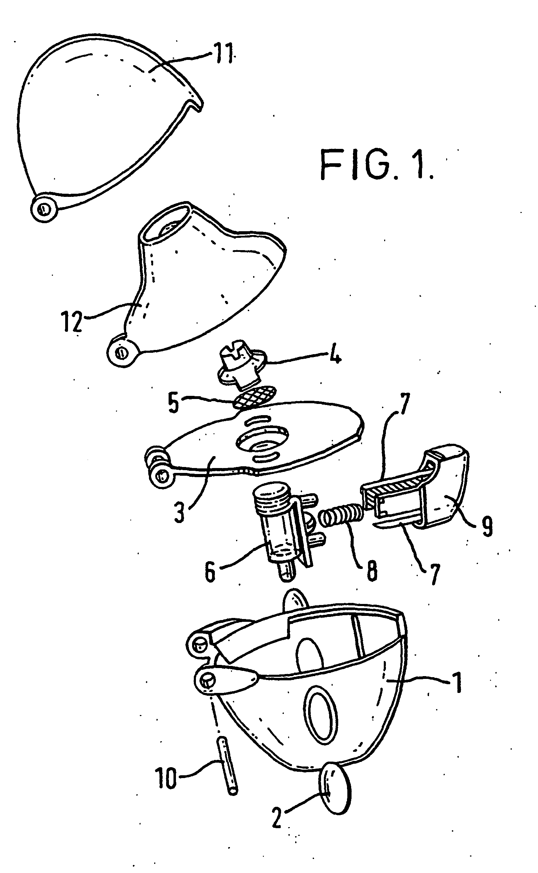

Ein zur Anwendung der erfindungsgemäßen Arzneimittelkombination in Inhaletten besonders bevorzugter Inhalator ist Figur 1 zu entnehmen.
Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 8 vorgesehen ist, sowie ein über eine Achse 10 klappbar mit dem Gehäuse 1, dem Deck 3 und einer Kappe 11 verbundenes Mundstück 12.

Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten erfindungsgemäß entweder gemeinsam oder jeweils die bereits vorstehend für **1'** und **2** genannten Dosierungen pro Einmalgabe.

### B) Treibgashaltige Inhalationsaerosole enthaltend die erfindungsgemäßen Wirkstoffkombinationen aus 1 und 2:

Erfindungsgemäße treibgashaltige Inhaltionsaerosole können **1** und **2** im Treibgas gelöst oder in dispergierter Form enthalten. Hierbei können **1** und **2** in getrennten. Darreichungsformen oder in einer gemeinsamen Darreichungsform enthalten sein, wobei **1** und **2** entweder beide gelöst, beide dispergiert oder jeweils nur eine Komponente gelöst und die andere dispergiert enthalten sein können.
Die zur Herstellung der erfindungsgemäßen Inhaltionsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die erfindungsgemäßen treibgashaltigen Inhaltionsaerosole können bis zu 5 Gew-% an Wirkstoff **1** und/oder **2** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2**.

Liegen die Wirkstoffe **1** und/oder **2** in dispergierter Form vor weisen die Wirkstoffteilchen bevorzugt eine mittlere Teilchengröße von bis zu 10 µm, bevorzugt von 0,1 bis 5 µm, besonders bevorzugt von 1 bis 5 µm auf.

Die vorstehend genannten erfindungsgemäßen treibgashaltigen Inhaltionaerosole können mittels im Stand der Technik bekannten Inhalatoren (MDIs = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, **dadurch gekennzeichnet, daß** sie vorstehend beschriebene erfindungsgemäße treibgashaltige Aerosole enthalten.
Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.

### C) Treibgasfreie Inhaltionslösungen oder Suspensionen enthaltend die erfindungsgemäßen Wirkstoffkombinationen aus 1 und 2:

Besonders bevorzugt erfolgt die Applikation der erfindungsgemäßen Wirkstoffkombination in Form von treibgasfreien Inhalationslösungen und Inhaltionssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 volumenprozent und besonders bevorzugt bei bis zu 30 Volumenprozent. Die restlichen Volumenprozente werden von Wasser aufgefüllt. Die **1** und **2** , getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.
Andere Ausführungsformen beinhalten diese Verbindung(en).
In einer solchen bevorzugten Ausführungsform liegt der Gehalt bezogen auf Natriumedetat unter 100 mg / 100 ml, bevorzugt unter 50 mg/100 ml, besonders beovorzugt unter 20 mg/ 100ml.
Generell sind solche Inhaltionslösungen bevorzugt, in denen der Gehalt an Natriumedetat bei 0 bis 10mg/100m1 liegt.

Den erfindungsgemäßen treibgasfreien Inhaltionslösungen können Co-Solventien und/oder weitere Hilfsstoffe zugesetzt werden.
Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der klein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünscht pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet

Zur Applikation der erfindungsgemäßen treibgasfreien Inhaltionslösungen sind besonders solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 20 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittel zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat® bekannt.

Dieser Vernebler (Respimat^{®}) kann vorteilhaft zur Erzeugung der erfindungsgemäßen inhalierbaren Aerosole enthaltend die Wirkstoffkombination aus **1** und **2** eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf die dort offenbarte Figur 1 und deren Beschreibung.
Der Düsenkörper besteht z.B. aus zwei fest miteinander verbundene Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schräubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sperrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Bestätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgebogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und genutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannt Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart.

Die Bauteile des Zerstäuber (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

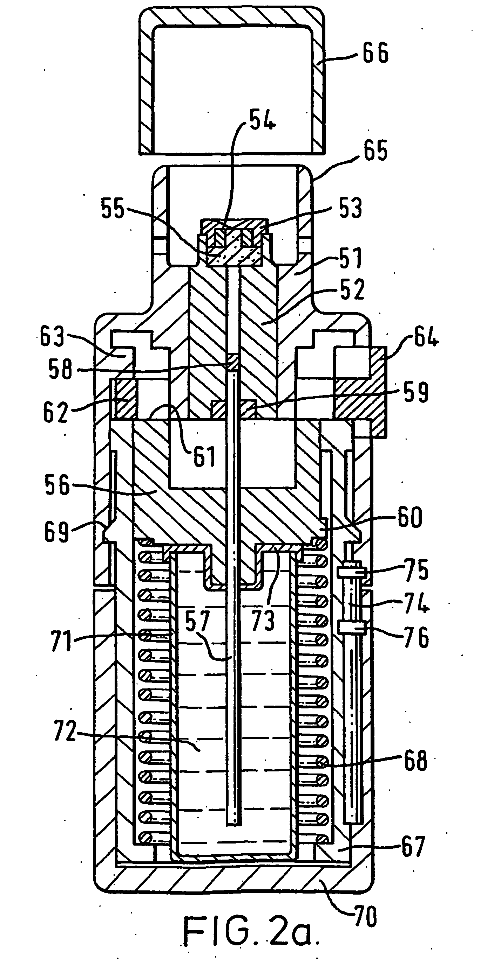

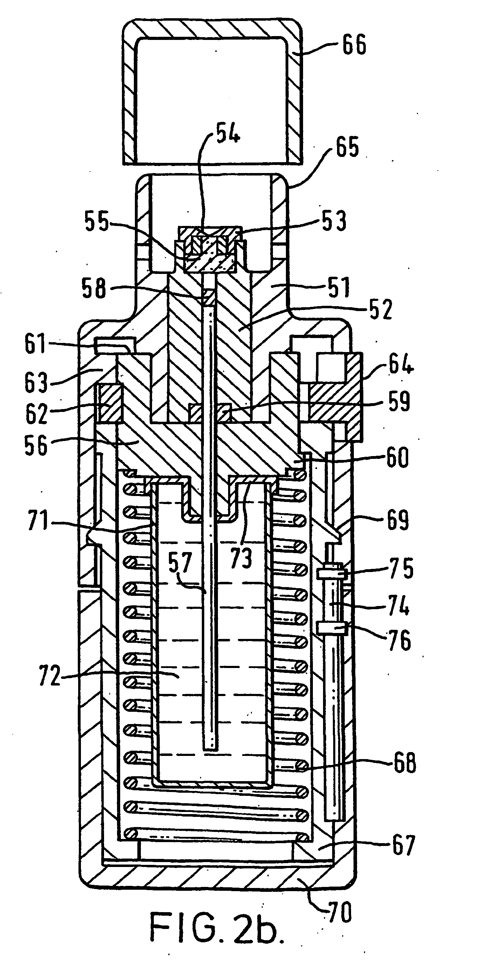

In den dieser Patentanmeldung beigefügten Figuren 2a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 2a zeigt einen Längsschnitt durch den Zerstäuber bei gespannter Feder, Figur 2b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuse hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73).verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat^{®}) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inahalatoren, beispielsweise Jet-Stream-Inhalatoren, vernebelt werden.

Dementsprechend betrifft ein weiterer Aspekt der vorliegenden Erfindung Arzneimittel in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat^{®}. Bevorzugt zielt die vorliegende Erfindung auf treibgasfreie Inhaltionslösungen oder Suspensionen gekennzeichnet durch die erfindungsgemäßen Wirkstoffkombination aus **1**. und **2** in Verbindung mit der unter der Bezeichnung Respimat^{®} bekannten Vorrichtung. Ferner betrifft die vorliegende Erfindung vorstehend genannte Vorrichtungen zur Inhalation, bevorzugt den Respimat^{®}, **dadurch gekennzeichnet, daß** sie vorstehend beschriebene erfindungsgemäße treibgasfreie Inhaltionslösungen oder Suspensionen enthalten..

Die erfindungsgemäßen treibgasfreien Inhalationslösungen oder Suspensionen können neben den vorstehend, zur Applikation im Respimat vorgesehenen Lösungen und Suspensionen auch als Konzentrate oder sterile gebrauchsfertige Inhalationslösungen bzw. -suspensionen vorlieben. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, **dadurch gekennzeichnet, daß** es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Ausgangsmaterialien

### Tiotropiumbromid:

Das in den nachfolgenden Formulierungsbeispielen eingesetzte Tiotropiumbromid kann wie in der Europäischen Patentanmeldung EP 418 716 A1 beschrieben, erhalten werden.

Zur Herstellung der erfindungsgemäßen Inhalationspulver kann ebenfalls kristallines Tiotropiumbromidmonohydrat eingesetzt werden. Dieses kristalline Tiotropiumbromidmonohydrat ist gemäß nachfolgend beschriebener Vorgehensweise erhältlich.
In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromidmonohydrat (86 % d. Th.)

Das so erhaltene kristalline Tiotropiumbromidmonohydrat wird nach bekannten Verfahren mikronisiert, um den Wirkstoff in Form der mittleren Teilchengröße bereitzustellen, die den erfindungsgemäßen Spezifikationen entspricht.

### Formulierungsbeispiele

Die nachfolgend unter A und B angegebenen inhaltiv applizierbaren Formulierungsbeispiele sind in Analogie zu im Stand der Technik bekannten Verfahren darstellbar.

### A) Inhaltionspulver:

1)

| **Bestandteile** | µ**g pro Kapsel** |
|---|---|
| Tiotropiumbromid | 21,7 |
| Epinastin-hydrochlorid | 200 |
| Lactose | 4778,3 |
| **Summe** | 5000 |

2)

| **Bestandteile** | µ**g pro Kapsel** |
|---|---|
| Tiotropiumbromid | 21,7 |
| Epinasfin-hydrochlorid | 125 |
| Lactose | 4853,3 |
| **Summe** | 5000 |

3)

| **Bestandteile** | µ**g pro Kapsel** |
|---|---|
| Tiotropiumbromid x H₂O | 22,5 |
| Epinastin-hydrochlorid | 250 |
| Lactose | 44727,5 |
| **Summe** | 5000 |

4)

| **Bestandteile** | **µg pro Kapsel** |
|---|---|
| Tiotropiumbromid | 21,7 |
| Epinastin-hydrochlorid | 250 |
| Lactose | 4728,3 |
| **Summe** | 5000 |

5)

| **Bestandteile** | **µg pro Kapsel** |
|---|---|
| Tiotropiumbromid x H₂O | 22,5 |
| Epinastin-hydrochlorid | 495 |
| Lactose | 4482,5 |
| **Summe** | 5000 |

6)

| **Bestandteile** | **µg pro Kapsel** |
|---|---|
| Tiotropiumbromid | 21,7 |
| Epinastin-hydrochlorid | 400 |
| Lactose | 4578,3 |
| **Summe** | 5000 |

### B) Treibgashaltige Inhaltionsaerosole:

1) Sirspensionsaerosol:

| **Bestandteile** | **Gew-%** |
|---|---|
| Tiotropiumbromid | 0,015 |
| Epinastin-hydrochlorid | 0,066 |
| Sojalecithin | 0,2 |
| TG134a : TG227 = 2:3 | ad 100 |

2) Suspensionsaerosol:

| **Bestandteile** | **Gew-%** |
|---|---|
| Tiotropiumbromid | 0,029 |
| Epinastin-hydrochlorid | 0,33 |
| Ethanol, absolut | 0,5 |
| Isopropylmyristat | 0,1 |
| TG 227 | ad 100 |

### C) Nasale Applikationsformen:

### 1) Lösung:

In einem geeigneten Gefäß werden 900 ml gereinigtes Wasser vorgelegt und unter Rühren 285.7 mg Tiötropiumbromid-monohydrat, 2000 mg Epinastinhydrochlorid und 500 mg Dinatrium EDTA gelöst. Anschließend wird mit 0.1 n Salzsäure der pH-Wert der Lösung auf pH 3 eingestellt und auf ein Gesamtvolumen von 1000 ml mit gereinigtem Wasser aufgefüllt. Die Lösung wird in eine geeignete Pumpe für die nasale Anwendung abgefüllt. Bei einem Hubvolumen von 70 µl pro Sprühstoß werden pro Anwendung 20 µg Tiotröpiumbromid und 140 µg Epinastinhydrochlorid verabreicht.

### 2 Pulver:

In einem geeigneten Mischer werden 20 g Tiotropiumbromidmonohydrat und 140 g Epinastinhydrochlorid mit einer Komgrößenvertellung für die beiden Wirkstoffe, die ca. 90% der Wirkstoffteilchen im Größenbereich 5 - 20 µm ausweist, vorgelegt. Zu den beiden Wirkstoffen werden 5.34 kg Lactose (200 M) hinzugegeben und anschließend solange gemischt, bis eine homogene Mischung entstanden ist. Es werden anschließend jeweils 5.5 mg dieser Mischung in eine nasales Pulversystem abgefüllt. Bei der nasalen Anwendung werden pro Hub 20 µg Tiotropiumbromid und 140 µg Epinastinhydrochlorid verabreicht.

## Patentansprüche

1. Arzneimittel **gekennzeichnet durch** einen Gehalt an Tiotropiumsalzen (1) in Kombination mit einem oder mehreren Antihistaminika (2), gegebenenfalls in Form ihrer Enantiomere, Gemische der Enantiomere oder in Form der Racemate, gegebenenfalls in Form der Solvate oder Hydrate sowie gegebenenfalls gemeinsam mit einem pharmazeutisch verträglichen Hilfsstoff, wobei es sich bei dem Antihistaminikum (**2**) um Epinastin, gegebenenfalls in Form seiner physiologisch verträglichen Säureadditionssalze handelt.

2. Arzneimittel nach Anspruch 1, **dadurch gekennzeichnet, daß 1** in Form des Chlorids, Bromids, Iodids, Methansulfonats, para-Toluolsulfonats oder Methylsulfats, bevorzugt in Form des Bromids enthalten ist.

3. Arzneimittel nach einem der Ansprüche 1 oder 2, **dadurch gekennzeichnet, daß 2** in Form eines seiner physiologisch verträglichen Säureadditionssalze zur Anwendung gelangt, die ausgewählt sind aus der Gruppe bestehend aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure.

4. Arzneimittel nach Anspruch 3, **dadurch gekennzeichnet, daß 2** in Form des Epinastinhydrochlorids zur Anwendung gelangt.

5. Arzneimittel nach einem der Ansprüche 1 bis 4, **dadurch gekennzeichnet, daß** die Gewichtsverhältnisse von **1** zu **2** in einem Bereich von 1:300 bis 50:1, bevorzugt von 1:250 bis 40:1 liegen.

6. Arzneimittel nach einem der Ansprüche 1 bis 5, **dadurch gekennzeichnet, daß** eine einmaliger Applikation einer Dosierung der Wirkstoffkombination **1** und **2** von 0,01 bis 10000µg, bevorzugt von 0,1 bis 2000µg entspricht.

7. Arzneimittel nach einem der Ansprüche 1 bis 6, **dadurch gekennzeichnet, daß** es in Form einer für die Inhalation oder die nasale Applikation geeigneten Darreichungsform vorliegt.

8. Arzneimittel nach Anspruch 7, **dadurch gekennzeichnet, daß** es sich um eine Darreichungsform ausgewählt aus der Gruppe Inhalationspulver, treibgashaltige Dosieraerosole und treibgasfreie Inhafationslösungen oder -suspensionen handelt.

9. Arzneimittel nach Anspruch 8, **dadurch gekennzeichnet, daß** es ein Inhalationspulver ist, welches **1** und **2** im Gemisch mit geeigneten physiologisch unbedenkliche Hilfsstoffen ausgewählt aus der Gruppe bestehend aus Monosaccharide, Disaccharide, Oligo- und Polysaccharide, Polyalkohole, Salze, oder Mischungen dieser Hilfsstoffe miteinandenthält.

10. Inhalationspulver nach Anspruch 9, **dadurch gekennzeichnet, daß** der Hilfsstoff eine maximale mittlere Teilchengröße von bis zu 250µm, bevorzugt zwischen 10 und 150µm aufweist.

11. Kapseln **gekennzeichnet durch** einen Gehalt an Inhaltionspulver nach Anspruch 9 oder 10.

12. Arzneimittel nach Anspruch 8, **dadurch gekennzeichnet, daß** es sich um ein treibgashaltiges Inhalationsaerosol handelt, welches **1** und **2** in gelöster oder dispergierter Form enthält.

13. Treibgashaltiges Inhalationsaerosol nach Anspruch 12, **dadurch gekennzeichnet, daß** es als Treibgas Kohlenwasserstoffe wie n-Propan, n-Butan oder Isobutan oder Halogenkohlenwasserstoffe wie chlorierte und/oder fluorierte Derivate des Methans, Ethans, Propans, Butans, Cyclopropans oder Cyclobutans enthält.

14. Treibgashaltiges Inhaltionsaerosol nach Anspruch 13, **dadurch gekennzeichnet, daß** das Treibgas TG134a, TG227 oder ein Gemisch davon darstellt.

15. Treibgashaltiges Inhalationsaerosol nach Anspruch 12, 13 oder 14, **dadurch gekennzeichnet, daß** es gegebenenfalls einen oder mehrere weitere Bestandteile ausgewählt aus der Gruppe bestehend aus Kosolventien, Stabilisatoren, oberflächenaktive Mittel (surfactants), Antioxidantien, Schmiermittel und Mittel zur Einstellung des pH-Werts enthält.

16. Treibgashaltiges Inhalationsaerosol nach einem der Ansprüche 12 bis 15, **dadurch gekennzeichnet, daß** es bis zu 5 Gew-% an Wirkstoff **1** und/oder **2** enthalten kann.

17. Arzneimittel nach Anspruch 8, **dadurch gekennzeichnet, daß** es sich um eine treibgasfreie Inhalationslösung oder -suspension handelt, die als Lösemittel Wasser, Ethanol oder ein Gemisch aus Wasser und Ethanol enthält.

18. Inhaltionslösung oder -suspension nach Anspruch 17, **dadurch gekennzeichnet, daß** der pH 2 - 7, bevorzugt 2 - 5 beträgt.

19. Inhaltionslösung oder -suspension nach Anspruch 18, **dadurch gekennzeichnet, daß** der pH mittels einer Säure ausgewählt aus der Gruppe bestehend aus Salzsäure, Bromwasserstoffsäure, Salpetersäure, Schwefelsäure, Ascorbinsäure, Zitronensäure, Äpfelsäure, Weinsäure, Maleinsäure, Bernsteinsäure, Fumarsäure, Essigsäure, Ameisensäure und Propionsäure oder Gemischen davon, eingestellt wird.

20. Inhalationslösung oder -suspension nach einem der Ansprüche 17 bis 19, **dadurch gekennzeichnet, daß** sie gegebenenfalls weitere Co-Solventien und/oder Hilfsstoffe enthält.

21. Inhalationslösung oder -suspension nach Anspruch 20, **dadurch gekennzeichnet, daß** sie als Co-Solventien Bestandteile enthält, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

22. Inhalationslösung oder -suspension nach einem der Ansprüche 20 oder 21, **dadurch gekennzeichnet, daß** sie als Hilfsstoffe oberflächenaktive Stoffe Stabilisatoren, Komplexbildner, Antioxidantien und/oder Konservierungsstoffe, Geschmackstoffe, pharmakologisch unbedenkliche Salze und/oder Vitamine enthält.

23. Inhalationslösung oder -suspension nach Anspruch 22, **dadurch gekennzeichnet, daß** sie als Komplexbildner, Editinsäure oder ein Salz der Editinsäure, bevorzugt Natriumedetat, enthält.

24. Inhalationslösung oder -suspension nach Anspruch 22 oder 23, **dadurch gekennzeichent, daß** sie als Antioxidantien,Verbindungen ausgewählt aus der Gruppe bestehend aus Ascorbinsäure, Vitamin A, Vitamin E und Tocopherole enthält.

25. Inhalationslösung oder -suspension nach Anspruch 22, 23 oder 24, **dadurch gekennzeichnet, daß** sie als Konservierungsmittel Verbindungen ausgewählt aus Cetylpyridiniumchlorid, Benzalkoniumchlorid, Benzoesäure und Benzoaten enthält.

26. Inhalationslösung oder -suspension nach einem der Ansprüche 20 bis 25, **dadurch gekennzeichnet, daß** sie neben den Wirkstoffen **1** und **2** und dem Lösemittel nur noch Bezalkoniumchlorid und Natriumedetat enthält.

27. Inhalationslösung oder -suspension nach einem der Ansprüche 20 bis 25, **dadurch gekennzeichnet, daß** sie neben den Wirkstoffen **1** und **2** und dem Lösemittel nur noch Benzalkoniumchlorid enthält.

28. Inhalationslösung oder -suspension nach einem der Ansprüche 17 bis 27, **dadurch gekennzeichnet, daß** es sich um ein Konzentrat oder eine sterile gebrauchsfertige Inhalationslösung oder -suspension handelt.

29. Verwendung einer Zusammensetzung nach einem der Ansprüche 1 bis 28 zur Herstellung eines Medikaments zur Behandlung von allergischer oder nicht allergischer Rhinitis.

## Claims

1. Medicament, **characterised in that** it contains tiotropium salts (**1**) combined with one or more antihistamines (**2**), optionally in the form of the enantiomers, mixtures of the enantiomers or in the form of the racemates thereof, optionally in the form of the solvates or hydrates and optionally together with a pharmaceutically acceptable excipient, the antihistamine (**2**) being epinastin, optionally in the form of the physiologically acceptable acid addition salts thereof.

2. Medicament according to claim 1, **characterised in that 1** is present in the form of the chloride, bromide, iodide, paratoluene sulphonate or methylsulphate, preferably in the form of the bromide.

3. Medicament according to one of claims 1 or 2, **characterised in that 2** is used in the form of one of the physiologically acceptable acid addition salts thereof which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid and maleic acid.

4. Medicament according to claim 3, **characterised in that 2** is used in the form of epinastin hydrochloride.

5. Medicament according to one of claims 1 to 4, **characterised in that** the weight ratios of **1** to **2** are in the range from 1:300 to 50:1, preferably from 1:250 to 40:1.

6. Medicament according to one of claims 1 to 5, **characterised in that** a single application corresponds to a dosage of the active substance combination **1** and **2** of 0.01 to 10000 µg, preferably from 0.1 to 2000 µg.

7. Medicament according to one of claims 1 to 6, **characterised in that** it is present in the form of a formulation suitable for inhalation or nasal administration.

8. Medicament according to claim 7, **characterised in that** it is a formulation selected from among inhalable powders, propellant-containing metered dose aerosols and propellant-free inhalable solutions or suspensions.

9. Medicament according to claim 8, **characterised in that** it is an inhalable powder, which contains **1** and **2** in admixture with suitable physiologically acceptable excipients selected from among the monosaccharides, disaccharides, oligo- and polysaccharides, polyalcohols, salts, or mixtures of these excipients with one another.

10. Inhalable powder according to claim 9, **characterised in that** the excipient has a maximum average particle size of up to 250 µm, preferably between 10 and 150 µm.

11. Capsules, **characterised in that** they contain an inhalable powder according to claim 9 or 10.

12. Medicament according to claim 8, **characterised in that** it is a propellant-containing inhalable aerosol which contains **1** and **2** in dissolved or dispersed form.

13. Propellant-containing inhalable aerosol according to claim 12, **characterised in that** it contains, as propellant gas, hydrocarbons such as n-propane, n-butane or isobutane or halohydrocarbons such as chlorinated and/or fluorinated derivatives of methane, ethane, propane, butane, cyclopropane or cyclobutane.

14. Propellant-containing inhalable aerosol according to claim 13, **characterised in that** the propellant gas is TG134a, TG227 or a mixture thereof.

15. Propellant-containing inhalable aerosol according to claim 12, 13 or 14, **characterised in that** it optionally contains one or more other ingredients selected from among cosolvents, stabilisers, surfactants, antioxidants, lubricants and means for adjusting the pH.

16. Propellant-containing inhalable aerosol according to one of claims 12 to 15, **characterised in that** it may contain up to 5 wt.-% of active substance **1** and/or **2**.

17. Medicament according to claim 8, **characterised in that** it is a propellant-fee inhalable solution or suspension which contains water, ethanol or a mixture of water and ethanol as solvent.

18. Inhalable solution or suspension according to claim 17, **characterised in that** the pH is 2 - 7, preferably 2 - 5.

19. Inhalable solution or suspension according to claim 18, **characterised in that** the pH is adjusted by means of an acid selected from among hydrochloric acid, hydrobromic acid, nitric acid, sulphuric acid, ascorbic acid, citric acid, malic acid, tartaric acid, maleic acid, succinic acid, fumaric acid, acetic acid, formic acid and propionic acid or mixtures thereof.

20. Inhalable solution or suspension according to one of claims 17 to 19, **characterised in that** it optionally contains other co-solvents and/or excipients.

21. Inhalable solution or suspension according to claim 20, **characterised in that** it contains as co-solvents ingredients which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters.

22. Inhalable solution or suspension according to one of claims 20 or 21, **characterised in that** it contains as excipients surfactants, stabilisers, complexing agents, antioxidants and/or preservatives, flavourings, pharmacologically acceptable salts and/or vitamins.

23. Inhalable solution or suspension according to claim 22, **characterised in that** it contains as complexing agent editic acid or a salt of editic acid, preferably sodium edetate.

24. Inhalable solution or suspension according to claim 22 or 23, **characterised in that** it contains, as antioxidants, compounds selected from among ascorbic acid, vitamin A, vitamin E and tocopherols.

25. Inhalable solution or suspension according to claim 22, 23 or 24, **characterised in that** it contains as preservatives compounds selected from cetyl pyridinium chloride, benzalkonium chloride, benzoic acid and benzoates.

26. Inhalable solution or suspension according to one of claims 20 to 25, **characterised in that** it contains, in addition to the active substances **1** and **2** and the solvent, only benzalkonium chloride and sodium edetate.

27. Inhalable solution or suspension according to one of claims 20 to 25, **characterised in that** it contains, in addition to the active substances **1** and **2** and the solvent, only benzalkonium chloride.

28. Inhalable solution or suspension according to one of claims 17 to 27, **characterised in that** it is a concentrate or a sterile ready-to-use inhalable solution or suspension.

29. Use of a composition according to one of claims 1 to 28 for preparing a medicament for treating allergic or non-allergic rhinitis.

## Revendications

1. Médicament **caractérisé par** une teneur en sels de tiotropium (**1**) en combinaison avec un ou plusieurs antihistaminiques (**2**), éventuellement sous forme de leurs énantiomères, de mélanges des énantiomères ou sous forme des racémates, éventuellement sous forme des solvates ou des hydrates et éventuellement avec un adjuvant pharmaceutiquement acceptable, l'antihistaminique (**2**) étant l'épinastine, éventuellement sous forme de ses sels d'addition d'acide physiologiquement acceptables.

2. Médicament selon la revendication 1 **caractérisé en ce que 1** est contenu sous forme du chlorure, bromure, iodure, méthanesulfonate, para-tolènesulfonate ou méthylsulfate, de préférence sous forme du bromure.

3. Médicament selon l'une des revendications 1 et 2 **caractérisé en ce que 2** est utilisé sous forme de l'un de ses sels d'addition d'acide physiologiquement acceptables, qui sont choisis dans le groupe consistant en les sels de l'acide chlorhydrique, de l'acide bromhydrique, de l'acide sulfurique, de l'acide phosphorique, de l'acide méthanesulfonique, de l'acide acétique, de l'acide fumarique, de l'acide succinique, de l'acide lactique, de l'acide citrique, de l'acide tartrique et de l'acide maléique.

4. Médicament selon la revendication 3 **caractérisé en ce que 2** est utilisé sous forme du chlorhydrate d'épinastine.

5. Médicament selon l'une des revendications 1 à 4 **caractérisé en ce que** les rapports massiques de **1** à **2** sont situés dans une plage de 1 : 300 à 50 : 1, de préférence de 1 : 250 à 40 : 1.

6. Médicament selon l'une des revendications 1 à 5 **caractérisé en ce qu'**une seule application correspond à un dosage de la combinaison de principes actifs **1** et **2** de 0,01 à 10000 µg, de préférence de 0,1 à 2000 µg.

7. Médicament selon l'une des revendications 1 à 6 **caractérisé en ce qu'**il est sous forme d'une forme d'administration appropriée à l'inhalation ou à l'application nasale.

8. Médicament selon la revendication 7 **caractérisé en ce qu'**il s'agit d'une forme d'administration choisie dans le groupe poudres pour inhalation, aérosols doseurs contenant un gaz propulseur et solutions ou suspensions pour inhalation sans gaz propulseur.

9. Médicament selon la revendication 8 **caractérisé en ce que** c'est une poudre pour inhalation qui contient **1** et **2** en mélange avec des adjuvants physiologiquement acceptables appropriés choisis dans le groupe consistant en les monosaccharides, les disaccharides, les oligo- et polysaccharides, les polyalcools, les sels ou les mélanges de ces adjuvants.

10. Poudre pour inhalation selon la revendication 9 **caractérisée en ce que** l'adjuvant a une granulométrie moyenne maximale pouvant atteindre 250 µm, de préférence entre 10 et 150 µm.

11. Capsules **caractérisées par** une teneur en poudre pour inhalation selon la revendication 9 ou 10.

12. Médicament selon la revendication 8 **caractérisé en ce qu'**il s'agit d'un aérosol pour inhalation contenant un gaz propulseur qui contient **1** et **2** sous forme dissoute ou dispersée.

13. Aérosol pour inhalation contenant un gaz propulseur selon la revendication 12 **caractérisé en ce qu'**il contient comme gaz propulseur des hydrocarbures comme le n-propane, le n-butane ou l'isobutane ou des hydrocarbures halogénés comme des dérivés chlorés et/ou fluorés du méthane, de l'éthane, du propane, du butane, du cyclopropane ou du cyclobutane.

14. Aérosol pour inhalation contenant un gaz propulseur selon la revendication 13 **caractérisé en ce que** le gaz propulseur est TG134a, TG227 ou un mélange de ceux-ci.

15. Aérosol pour inhalation contenant un gaz propulseur selon la revendication 12, 13 ou 14 **caractérisé en ce qu'**il contient éventuellement un ou plusieurs autres constituants choisis dans le groupe consistant en les cosolvants, les stabilisants, les agents tensioactifs (surfactants), les antioxydants, les lubrifiants et les agents pour le réglage du pH.

16. Aérosol pour inhalation contenant un gaz propulseur selon l'une des revendications 12 à 15 **caractérisé en ce qu'**il peut contenir jusqu'à 5 % en masse de principe actif **1** et/ou **2**.

17. Médicament selon la revendication 8 **caractérisé en ce qu'**il s'agit d'une solution ou suspension pour inhalation sans gaz propulseur qui contient comme solvant de l'eau, de l'éthanol ou un mélange d'eau et d'éthanol.

18. Solution ou suspension pour inhalation selon la revendication 17 **caractérisée en ce que** le pH est 2 - 7, de préférence 2 - 5.

19. Solution ou suspension pour inhalation selon la revendication 18 **caractérisée en ce que** le pH est réglé au moyen d'un acide choisi dans le groupe consistant en l'acide chlorhydrique, l'acide bromhydrique, l'acide nitrique, l'acide sulfurique, l'acide ascorbique, l'acide citrique, l'acide malique, l'acide tartrique, l'acide maléique, l'acide succinique, l'acide fumarique, l'acide acétique, l'acide formique et l'acide propionique ou leurs mélanges.

20. Solution ou suspension pour inhalation selon l'une des revendications 17 à 19 **caractérisée en ce qu'**elle contient éventuellement d'autres cosolvants et/ou adjuvants.

21. Solution ou suspension pour inhalation selon la revendication 20 **caractérisée en ce qu'**elle contient comme cosolvants des constituants qui contiennent des groupes hydroxyle ou d'autres groupes polaires, par exemple des alcools - en particulier l'alcool isopropylique, des glycols - en particulier le propylèneglycol, le polyéthylèneglycol, le polypropylèneglycol, l'éther de glycol, le glycérol, des alcools polyoxyéthylénés et des esters d'acides gras polyoxyéthylénés.

22. Solution ou suspension pour inhalation selon l'une des revendications 20 et 21 **caractérisée en ce qu'**elle contient comme adjuvants des substances tensioactives, des stabilisants, des complexants, des antioxydants et/ou des conservateurs, des agents de sapidité, des sels pharmacologiquement acceptables et/ou des vitamines.

23. Solution ou suspension pour inhalation selon la revendication 22 **caractérisée en ce qu'**elle contient comme complexant l'acide édétique ou un sel de l'acide édétique, de préférence l'édétate de sodium.

24. Solution ou suspension pour inhalation selon la revendication 22 ou 23 **caractérisée en ce qu'**elle contient comme antioxydants des composés choisis dans le groupe consistant en l'acide ascorbique, la vitamine A, la vitamine E et les tocophérols.

25. Solution ou suspension pour inhalation selon la revendication 22, 23 ou 24 **caractérisée en ce qu'**elle contient comme conservateurs des composés choisis parmi le chlorure de cétylpyridinium, le chlorure de benzalkonium, l'acide benzoïque et les benzoates.

26. Solution ou suspension pour inhalation selon l'une des revendication 20 à 25 **caractérisée en ce qu'**elle contient, outre les principes actifs **1** et **2** et le solvant, seulement du chlorure de benzalkonium et de l'édétate de sodium.

27. Solution ou suspension pour inhalation selon l'une des revendication 20 à 25 **caractérisée en ce qu'**elle contient, outre les principes actifs **1** et **2** et le solvant, seulement du chlorure de benzalkonium.

28. Solution ou suspension pour inhalation selon l'une des revendication 17 à 27 **caractérisée en ce qu'**il s'agit d'un concentré ou d'une solution ou suspension pour inhalation prête à l'emploi stérile.

29. Utilisation d'une composition selon l'une des revendications 1 à 28 pour la production d'un médicament pour le traitement de la rhinite allergique ou non allergique.
